(19) Europäisches Patentamt
European Patent Office
Office européen des brevets

(11) EP 4 650 379 A1

# (12) EUROPEAN PATENT APPLICATION
published in accordance with Art. 153(4) EPC

(43) Date of publication:
**19.11.2025 Bulletin 2025/47**

(21) Application number: **23944144.7**

(22) Date of filing: **14.09.2023**

(51) International Patent Classification (IPC):
***C08G 18/79** (2006.01)*

(52) Cooperative Patent Classification (CPC):
**C08G 18/79; C09D 175/04**

(86) International application number:
**PCT/CN2023/118777**

(87) International publication number:
**WO 2025/007421 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.07.2023 CN 202310821678**

(71) Applicant: **Wanhua Chemical Group Co., Ltd.**
**Yantai, Shandong 264006 (CN)**

(72) Inventors:
- **LIU, Wei**
  **Yantai, Shandong 264006 (CN)**
- **REN, Yizhen**
  **Yantai, Shandong 264006 (CN)**
- **LIN, Chengdong**
  **Yantai, Shandong 264006 (CN)**
- **SHI, Bin**
  **Yantai, Shandong 264006 (CN)**
- **SHANG, Yonghua**
  **Yantai, Shandong 264006 (CN)**
- **YANG, Zhimin**
  **Yantai, Shandong 264006 (CN)**
- **ZHANG, Xianfeng**
  **Yantai, Shandong 264006 (CN)**
- **TAN, Jun**
  **Yantai, Shandong 264006 (CN)**
- **QIAO, Xiaofei**
  **Yantai, Shandong 264006 (CN)**
- **LI, Yuan**
  **Yantai, Shandong 264006 (CN)**

(74) Representative: **Potter Clarkson**
**Chapel Quarter**
**Mount Street**
**Nottingham NG1 6HQ (GB)**

## (54) POLYISOCYANATE COMPOSITION

(57) Disclosed in the present disclosure is a polyisocyanate composition, which comprises an isocyanurate (methyl-substituted IPDI trimer) having a structure A and an isocyanurate (IPDI trimer) having a structure B. by means of limiting the molar ratio of the two isocyanurates, the solubility of the polyisocyanate composition in a non-polar solvent is enhanced, making it convenient for downstream customers to use the polyisocyanate composition.

EP 4 650 379 A1

## Description

### TECHNICAL FIELD

The embodiments of the present application relate to the technical field of polyisocyanates, such as a polyisocyanate composition.

### BACKGROUND

The technology of modifying isophorone diisocyanate (IPDI) monomers under the action of catalysts is well known in the art, such as a polymerization reaction or an alcohol modification reaction. After achieving the expected conversion rate, unreacted IPDI monomers are removed by vacuum distillation or thin film evaporation to obtain an IPDI-based polyisocyanate composition product. Reference can be made to US2004176562A1, US6093817, CN107827832, etc.

The polyisocyanate composition prepared by IPDI polymerization has obvious quick drying characteristics and has been widely used in the polyurethane coating industry. Moreover, because the polyisocyanate composition prepared by IPDI is mostly solid at room temperature, it is a common practice for customers in the industry to dissolve it in solvents for use.

In the actual downstream application process, it was found that the IPDI-based polyisocyanate composition has poor solubility in non-polar solvents, and the turbidity is prone to increase, which in turn affects the use of downstream customers.

### SUMMARY OF THE INVENTION

The following is an overview of a subject described in detail herein. This overview is not intended to limit the scope of protection of the claims.

In an actual research process, researchers have found that presence of a following methyl-substituted specific structure A in a polyisocyanate composition increases solubility of the composition in a non-polar solvent.

In order to enhance the solubility of the non-polar solvent, the technical solution adopted in the examples of the present application is as follows:

a polyisocyanate composition includes isocyanurate having a structure A and isocyanurate having a structure B, wherein the structure A is:

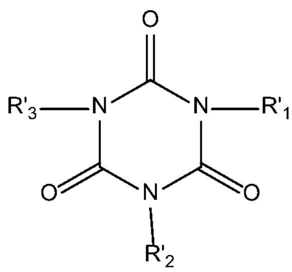

(A)

$R'_1$, $R'_2$ and $R'_3$ are respectively selected from the following four structures, wherein at least one of $R'_1$, $R'_2$ and $R'_3$ is selected from a structure (I) or a structure (II);

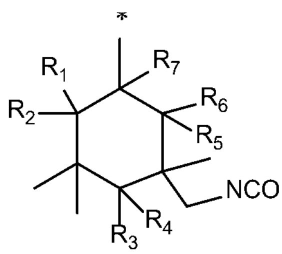

(I),

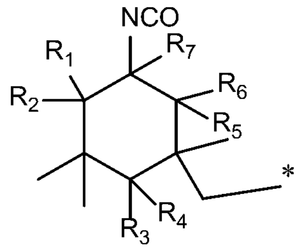

(II),

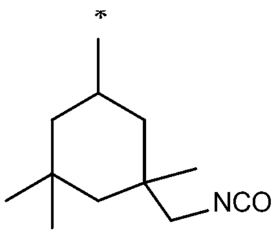

(III),

and

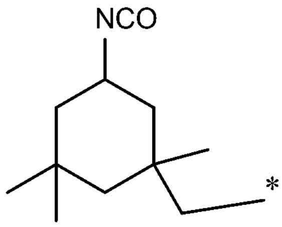

(IV);

and

one, two, or three group(s) among $R_1$, $R_2$, $R_3$, $R_4$, $R_5$, $R_6$ and $R_7$ in the above structural formulae is/are $CH_3$, and the rest are hydrogen.

The structure B is

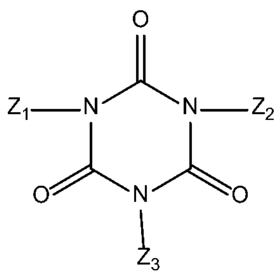

.

$Z_1$, $Z_2$ and $Z_3$ may be any one or both of the following two structures:

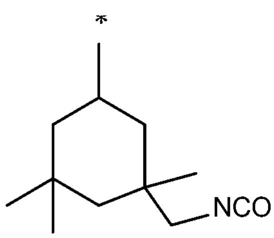

(III)

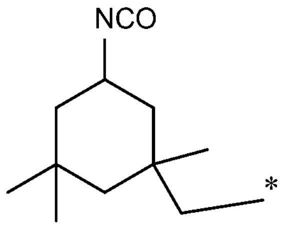

(IV)

Meanwhile, a molar ratio of the above isocyanurate having the structure A to the above isocyanurate having the structure B is in a range from 0.0005 to 0.03.

According to the polyisocyanate composition described in the present application, the molar ratio of the isocyanurate having the structure A to the isocyanurate having the structure B is in a range from 0.005 to 0.02.

The molar ratio of the isocyanurate having the structure A to the isocyanurate having the structure B enhances its solubility in the non-polar solvent when being above a lower limit, which is beneficial for a paint film, and inhibits increase in viscosity of a solution state when being below an upper limit, which is beneficial for downstream construction.

In the present application, the structure B is an isophorone diisocyanate trimer, and the structure A is a methyl-substituted isophorone diisocyanate trimer.

A control manner for the molar ratio of the isocyanurate having the structure A to the isocyanurate having the structure B is not specifically limited, as long as it can achieve the purpose. For example, the molar ratio of the present application may be directly obtained by polymerizing isophorone diisocyanate or achieved by adding the structure A.

If the composition is prepared by polymerizing isophorone diisocyanate, contents of the structure A and the structure B in the final composition may be controlled by controlling a content of methyl-substituted isophorone diisocyanate in raw material isophorone diisocyanate.

Wherein, the structure of the methyl-substituted isophorone diisocyanate (methyl-substituted IPDI) is as follows:

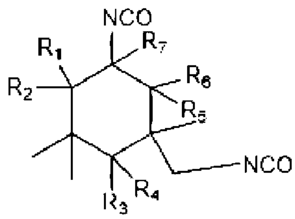

wherein, $R_1$, $R_2$, $R_3$, $R_4$, $R_5$, $R_6$, and $R_7$ have the same meaning as Formula I and Formula II.

The content of the methyl-substituted isophorone diisocyanate may be adjusted during the preparation of isophorone diisocyanate to obtain an isophorone diisocyanate monomer containing the methyl-substituted isophorone diisocyanate, and may also be controlled in an addition manner before the preparation of the trimer.

A preparation process of IPDI: isophorone nitrile is prepared from isophorone and hydrogen cyanide, and then further reacts with ammonia and hydrogen to generate isophorone diamine, and the isophorone diamine is subjected to a phosgenation process to obtain the IPDI. Through research, it has been found that a methyl-substituted IPDI monomer may be generated in the preparation process of the IPDI. A content of methyl-substituted products in the IPDI may be controlled by controlling reaction and/or separation conditions, such as controlling a methylamine content in raw material ammonia, a chloride content in a hydrogenation catalyst, or the number of rectification times and rectification conditions. The methyl-substituted isophorone diisocyanate trimer (structure A) may also be prepared separately, and the molar ratio of the two can be adjusted in an addition manner.

The specific preparation method of the IPDI containing the methyl-substituted isophorone diisocyanate may refer to CN115894857A.

In the present application, according to the polyisocyanate composition based on the isophorone diisocyanate, a mass content of the isocyanurate having the structure B is in a range from 50% to 75%, preferably in a range from 50% to 70%, more preferably in a range from 55% to 65%, and more preferably in a range from 59% to 63%. The content may be determined by gel chromatography.

In the present application, according to the polyisocyanate composition based on the isophorone diisocyanate, a mass content of isocyanate groups (-NCO) is in a range from 16% to 18%, preferably in a range from 16.5% to 17.5%. On the one hand, an NCO group content in the composition should not be too low, and the too low content is not conducive to structural cross-linking of the paint film. On the other hand, the NCO content should not be too high, and the too high content usually reduces the hardness of the paint film.

In the present application, the polyisocyanate composition based on the isophorone diisocyanate may be solid or

a solution state after being dissolved in a solvent.

Due to the fact that the polyisocyanate composition described in the present application is in a solid state at room temperature, generally at 25°C. In some specific implementations, the polyisocyanate composition based on the isophorone diisocyanate described in the present application may also be dissolved in a solvent to obtain a polyisocyanate composition solution, which appears as a solution state after being dissolved in the solvent.

In the polyisocyanate composition solution of the present application, the solvent is selected from any one or a combination of at least two of butyl acetate, ethyl acetate, solvent oil, toluene, xylene, propylene glycol methyl ether acetate, and 2-heptanone.

Preferably, a mass proportion of the solvent is in a range from 28% to 32%, such as 29%, 30%, and 31%, preferably 30%, based on 100% of total mass of the polyisocyanate composition solution. If the solvent content in the composition solution is too high, the economic benefits of downstream applications are reduced, while if the solvent content is too low, the viscosity of the solution will be too high, which is not beneficial for downstream use.

Preferably, the polyisocyanate composition solution corresponding to the solution state obtained by dissolving the same in the solvent has an NCO group mass content ranging from 11.2% to 12.8%, such as 11.3%, 11.6%, 11.9%, 12.2%, 12.5% and 12.8%, preferably 12%, based on 100% of the total mass of the polyisocyanate composition solution.

In some specific implementations, in the polyisocyanate composition based on the isophorone diisocyanate described in the present application, a content of an isophorone diisocyanate monomer is less than 0.5wt%, such as 0.01wt%, 0.05wt%, 0.1wt%, 0.2wt%, 0.3wt% and 0.4wt%, preferably less than 0.35wt%, and more preferably less than 0.1wt%. The decrease of the content of the IPDI monomer is beneficial for downstream construction safety.

A method of polymerizing the isophorone diisocyanate is not limited, as long as it can make the components of the polyisocyanate composition meet the aforementioned requirements of the present application.

According to the method disclosed in the related art, for example, the polyisocyanate composition based on the isophorone diisocyanate may be specifically obtained by using following methods:

in the presence of a polymerization catalyst, isophorone diisocyanate undergoes a polymerization reaction.

The polymerization reaction is terminated when the mass content of NCO groups in a system is in a range from 20% to 30%, such as 24%, 25%, 26%, and 27%. Then, unreacted monomers are removed to obtain the polyisocyanate composition based on the isophorone diisocyanate.

A basic method for the polymerization reaction of isocyanate is known in the related art. In the specific polymerization reaction process, isophorone diisocyanate may be added to a round-bottom flask equipped with a reflux condenser, a stirrer, a thermometer, and a nitrogen inlet. Then, the system is heated to a predetermined reaction temperature range (such as 50°C-110°C, for example, 70°C and 90°C), and then a catalyst solution is added dropwise. As is well known to those skilled in the art, the specific operating parameters and sequence of the polymerization reaction are common knowledge in the art and will not be repeated here.

In some specific implementations, the use amount of a catalyst is 0.001%-0.1% of the mass of isophorone diisocyanate, such as 0.005%, 0.01%, 0.02%, 0.04%, 0.06%, 0.08%, preferably 0.01%-0.03%.

The polymerization catalyst used in the present application is selected from: (1) hydroxides of tetraalkylammonium (such as tetramethylammonium hydroxide, tetraethylammonium hydroxide, tetrabutylammonium hydroxide, and benzyltrimethylammonium hydroxide), or organic acid salts of tetraalkylammonium (such as tetramethylammonium acetate, and tetraethylammonium butyrate); (2) hydroxides of hydroxyalkylammonium (such as trimethylhydroxypropylammonium, trimethylhydroxyethyl ammonium, and triethylpropylammonium) or organic acid (such as acetic acid, butyric acid, and isooctanoic acid) salts of hydroxyalkylammonium; or (3) metal (such as tin, and zinc) salts of alkyl carboxylic acid (such as acetic acid, decanoic acid, and octanoic acid); preferably one or more of (1) or (2).

In some specific implementations, the catalyst used in the present application is dissolved in an organic solvent to be prepared into the catalyst solution, and the catalyst solution is then added to the system to catalyze the polymerization reaction.

Specifically, in the catalyst solution, a mass concentration of the catalyst is in a range from 1% to 40%, such as 5%, 10%, 15%, 20%, 25%, 30%, and 35%, preferably in a range from 10% to 30%.

Specifically, the organic solvent is selected from alcohol solvents, preferably any one or a combination of at least two of monoalcohol and diol. In the present application, the monoalcohol may be selected from any one or a combination of at least two of 1-propanol, 2-propanol, n-butanol, isobutanol, sec butanol, tert butanol, n-octanol, isooctanol, heptanol, n-butanol, hexanol, heptanol, or isooctanol. The diol may be selected from any one or a combination of at least two of diethylene glycol, 1,3-butanediol, 1,6-hexanediol, 1,4-butanediol, 2-ethyl-1,6-hexanediol, etc.

In some specific implementations, when the above polymerization reaction proceeds to an NCO value of 23%-28% in the system, such as 24%, 25%, 26%, 27%, a terminating agent may be used to terminate the reaction. Specifically, a ratio of the use amount of the terminating agent to the molar weight of the polymerization catalyst is in a range from 1 to 1.1, such as 1.02, 1.04, 1.06, and 1.08, preferably 1.

The terminating agent is selected from any one or a combination of at least two of phosphate ester terminating agents or benzenesulfonic acid terminating agents, such as any one or a combination of at least two of dibutyl phosphate,

diisooctyl phosphate, or p-toluenesulfonic acid.

In some other implementations, the above polymerization reaction may also be terminated by heating and staying for a period of time. In the specific implementation process, the system may be heated to 120°C-130°C (such as 122°C, 124°C, 126°C, and 128°C) and stayed for 30-60 minutes (such as 35 minutes, 40 minutes, 45 minutes, 50 minutes, and 55 minutes).

In some specific implementations, an optional technical solution is to control the mass content of the methyl-substituted IPDI in the raw material IPDI monomer to be in a range from 0.01% to 0.27%, obtain a reaction solution under the above reaction conditions, and then remove the unreacted isophorone diisocyanate monomer in the polymerization reaction system through thin film evaporation. In some specific implementations, a thin film evaporator may be used to remove the unreacted isophorone diisocyanate monomer in the reaction system under the conditions of 170°C-200°C and 10 PaA-200 PaA (such as 50 PaA, 100 PaA, and 150 PaA), and obtain the polyisocyanate composition of the present application.

Another optional technical solution is to directly perform the polymerization reaction on the methyl-substituted IPDI obtained by enrichment according to the above method to obtain a polyisocyanate composition rich in the structure A. The polyisocyanate composition of the present application can also be obtained by blending the composition rich in the structure A with a polyisocyanate composition rich in the structure B.

The polyisocyanate composition based on the IPDI described in the present application may be used in fields such as automotive repair paint, automotive factory paint, and rail transit coatings.

Compared with the related art, the beneficial effects of the technical solutions of the examples of the present application lie in that a solvent type of the non-polar solvent may be increased while ensuring appropriate viscosity for downstream construction, thereby facilitating the use of downstream customers.

After reading and understanding the detailed description, other aspects can be understood.

## DETAILED DESCRIPTION

In order to better understand the present application, the following will further explain the content of the present application in conjunction with examples.

Source information of main raw materials used in the following examples is as follows. Unless otherwise specified, the main raw materials are all ordinary commercially available raw materials.

Source information of main raw materials used in the following examples is as follows. Unless otherwise specified, the main raw materials are all ordinary commercially available raw materials:

isophorone diisocyanate (IPDI): WANHUA CHEMICAL GROUP CO., LTD. ;

n-hexanol: sigma-Aldrich, 98%;

isooctanol: sigma-Aldrich, 98%;

n-butanol: sigma-Aldrich, 99%;

1,3-butanediol: sigma-Aldrich, 99%;

trimethylhydroxypropyl ammonium isooctanoate: sigma-Aldrich, 95%;

benzyltrimethylammonium hydroxide: sigma-Aldrich, 95%;

tetra-n-butylammonium hydroxide: sigma-Aldrich, 95%;

tetrabutyl ammonium acetate: sigma-Aldrich, 96%;

dibutyl phosphate: sigma-aldrich, 97%;

diisooctyl phosphate: sigma-aldrich, 96%;

p-toluenesulfonic acid: sigma-aldrich, 95%;

butyl acetate: Sinopharm Chemical Reagent Co., Ltd., 99%; and

solvent oil S100: industrial product of Huafon Chemical Co., Ltd.

The main testing methods used in each example of the present application are as follows:

1. Method for testing a content of monomers in a polyisocyanate composition: GB/T 18446-2009 is adopted.

2. Measurement of NCO group content: the NCO group content based on total mass of a sample is obtained by neutralizing isocyanate groups in a test specimen with an excess of 2mol/L di-n-butylamine and performing back titration with 1mol/L hydrochloric acid. The calculation formula is as follows:

$$NCO = \frac{(V_0 - V) \times C \times 0.04202}{m} \times 100$$

In the formula:

V means the use amount of a hydrochloric acid standard solution at the time of titration of the specimen, ml;
V0 means the use amount of the hydrochloric acid standard solution at the time of titration blank, ml;
C means actual concentration of the hydrochloric acid standard solution, mol/L;
0.04202 means mass of isocyanate ions expressed in grams equivalent to 1.00ml hydrochloric acid standard solution [c(HCl)=1.000mol/L]; and
M means total mass of the sample, g.

3. Method for testing a content of methyl-substituted IPDI in an IPDI monomer

The content of methylated isophorone diisocyanate in the isophorone diisocyanate composition described in the present application may be analyzed by gas chromatography injection: the sample is dissolved in a solvent (preferably dichloromethane) and analyzed by gas chromatography injection, detected by a flame ionization detector (FID), and quantitatively calculated by an area normalization method.

The chromatographic conditions are as follows:

carrier gas: purified and dried high-purity nitrogen (purity≥99.999%);
combustion gas: hydrogen gas (purity ≥ 99.999%), flow rate of 40 mL/min;
combustion-supporting gas: purified and dried air, flow rate of 400 mL/min;
purge gas: nitrogen, flow rate of 30 mL/min;
column flow rate: 1.06 mL/min;
split ratio: 30:1;
column temperature (programmed heating): being kept at 140°C for 0 minute, heated up to 220°C at 10°C/min, kept for 1 minute, heated up to 260°C at 5°C/min, kept for 0 minute, heated up to 280°C at 10°C/min, and kept for 1 minute;
injection port temperature: 270°C;
detector temperature: 290°C; and
injection volume: 0.2 μL.

4. Molar ratio of structure A to structure B:

A molar ratio of isocyanurate structure formed by polymerization of methyl substituted IPDI relative to isocyanurate trimers (referred to as compound α) formed by polymerization of three IPDIs in the polyisocyanate composition is calculated using the following method.

Specifically, the terminal isocyanate groups of the polyisocyanate composition of this patent application are subjected to carbamation with methanol and analyzed using liquid chromatography-mass spectrometry (LC/MS). The following records preparation and measurement methods of the sample.

1) Preparation method of the sample

100 mg of polyisocyanate composition is weighed, and methanol is added to make the amount be 10 mg/mL. Then, a mixture undergoes standing for 2 days to allow the existing isocyanate groups to completely react with methanol, and a methanol solution is prepared.

2) Measurement method

The methanol solution obtained above is measured using the following device.

**·LC**

Device: Waters ACQUITY UPLC
Column: Phenomenex, Kinetex 2.6μXB-C18 100A (inner diameter of 2.1 mm, length of 50 mm)
Column temperature: 40°C
Detection: 220 nm
Flow rate: 0.3 mL/min
Mobile phase: gradient of A and B solutions, A=water (0.05% formic acid), B=acetonitrile (0.05% formic acid)
Injection volume: 1 μL

**·MS**

Device: Waters, Synapt G2

Ionization: ESI

Mode: Positive

Scanning range: m/z 250-2000

A methanol adduct (compound β) of isocyanurate trimer formed by polymerization of 3 IPDIs is detected by detecting ion (m/z) 762.
For the isocyanurate structure formed by the polymerization of the methyl-substituted IPDI, the difference in the number of methyl substitutions leads to differences in the ion (m/z) position.
The compounds, in which ion peak positions (m/z) of the isocyanurate structure formed by polymerization of 1, 2, or 3 methyl-substituted IPDI(s) are 776, 790, and 804, are defined as compounds a, b, and c;
The compounds, in which ion peak positions (m/z) of the isocyanurate structure formed by polymerization of 4, 5, or 6 methyl-substituted IPDIs are 818, 832, and 846, are defined as compounds d, e, and f; and
The compounds, in which ion peak positions (m/z) of the isocyanurate structure formed by polymerization of 7, 8, or 9 methyl-substituted IPDIs are 860, 874, and 888, are defined as compounds g, h, and i.
A ratio of a peak area of compounds a to i to a peak area of the compound β is calculated as the molar ratio of the structure A to the structure B.

5. Evaluation method for solubility of non-polar solvent

The polyisocyanate composition is mixed with the solvent oil S100 in a mass ratio of 1:1 to obtain a diluent, and then a turbidity meter HACH 2100 is used for testing:

turbidity not higher than 0.3 indicates excellent solubility;
turbidity greater than 0.3 but not higher than 0.6 indicates good solubility;
turbidity greater than 0.6 but not higher than 0.9 indicates acceptable solubility; and
turbidity greater than 0.9 indicates poor solubility.

6. Evaluation method for dissolution viscosity of polar solvent

The polyisocyanate composition is mixed with butyl acetate in a mass ratio of 7:3 to obtain a solution product, and 25°C viscosity test is conducted using a Broolfield DV2T viscometer:

25°C viscosity not higher than 600 cP is excellent for downstream construction;
25°C viscosity not higher than 800 cP is good for downstream construction;
25°C viscosity not higher than 1000 cP is medium grade for downstream construction; and
25°C viscosity greater than 1000 cP is poor for downstream construction.

7. Method for testing solid content:

the precise amount of a composition solution sample (about 0.5g) is weighed and placed in an aluminum foil dish (accurately weighed by mass). The aluminum foil dish is placed in a 120°C oven and undergoes standing for 2 hours. The aluminum foil dish is taken out and weighed after cooling.

Solid content = (weight after drying - weight of aluminum foil dish) / sample weight * 100%.

### Example 1

Preparation of catalyst solution: tetra-n-butylammonium hydroxide was dissolved in n-butanol to prepare a solution with a concentration of 20wt%.

1000 g of IPDI (a mass content of methyl-substituted IPDI being 0.01%) was placed in a round-bottom flask equipped with a reflux condenser, a stirrer, a thermometer, and a nitrogen inlet; the above reaction system was heated to 70°C, and then the catalyst solution prepared above was added dropwise to a reaction system while stirring (the use amount of catalyst is 0.015% of IPDI mass); a reaction temperature was controlled between 70°C and 80°C for a polymerization reaction; and when the NCO mass content of the reaction system is 28%, dibutyl phosphate with the same molar amount as the catalyst was added to terminate the reaction.

A thin film evaporator was used to evaporate and remove unreacted isophorone diisocyanate monomers from a polymerization reaction solution under conditions of a temperature of 185°C and an absolute pressure of 50 Pa, with a staying time of 10 minutes, so that a content was 0.34wt%; and an IPDI trimer (i.e. isocyanate having a structure B) with a mass content of 68% was dissolved in butyl acetate to obtain a solution product with a solid content of 70wt%, and a polyisocyanate composition solution 1 was obtained.

### Example 2

Preparation of catalyst solution: trimethylhydroxypropyl ammonium isooctanoate was dissolved in isooctanol to prepare a solution with a concentration of 30wt%.

1000 g of IPDI (a mass content of methyl-substituted IPDI being 0.08%) was placed in a round-bottom flask equipped with a reflux condenser, a stirrer, a thermometer, and a nitrogen inlet; the above reaction system was heated to 80°C, and then the catalyst solution prepared above was added dropwise to a reaction system while stirring (the use amount of catalyst is 0.016% of IPDI mass); a reaction temperature was controlled between 80°C and 90°C for a polymerization reaction; and when the NCO mass content of the reaction system is 25%, diisooctyl phosphate with the same molar amount as the catalyst was added to terminate the reaction.

A thin film evaporator was used to evaporate and remove unreacted isophorone diisocyanate monomers from a polymerization reaction solution under conditions of a temperature of 185°C and an absolute pressure of 50 Pa, with a staying time of 15 minutes, so that a content was 0.32wt%; and an IPDI trimer (i.e. isocyanate having a structure B) with a mass content of 62% was dissolved in butyl acetate to obtain a solution product with a solid content of 70wt%, and a polyisocyanate composition solution 2 was obtained.

### Example 3

Preparation of catalyst solution: benzyltrimethylammonium hydroxide was dissolved in n-hexanol to prepare a solution with a concentration of 1wt%.

1000 g of IPDI (a mass content of methyl-substituted IPDI being 0.18%) was placed in a round-bottom flask equipped with a reflux condenser, a stirrer, a thermometer, and a nitrogen inlet; the above reaction system was heated to 60°C, and then the catalyst solution prepared above was added dropwise to a reaction system while stirring (the use amount of catalyst is 0.015% of IPDI mass); a reaction temperature was controlled between 60°C and 70°C for a polymerization reaction; and when the NCO mass content of the reaction system is 25%, diisooctyl phosphate with a molar ratio of 1.1 to the catalyst was added to terminate the reaction.

A thin film evaporator was used to evaporate and remove unreacted isophorone diisocyanate monomers from a polymerization reaction solution under conditions of a temperature of 200°C and an absolute pressure of 100 Pa, with a staying time of 10 minutes, so that a content was 0.33wt%; and an IPDI trimer (i.e. isocyanate having a structure B) with a mass content of 62% was dissolved in butyl acetate to obtain a solution product with a solid content of 70wt%, and a polyisocyanate composition solution 3 was obtained.

**Example 4**

Preparation of catalyst solution: tetrabutyl ammonium acetate was dissolved in 1,3-butanediol to prepare a solution with a concentration of 10wt%.

1000 g of IPDI (a mass content of methyl-substituted IPDI being 0.35%) was placed in a round-bottom flask equipped with a reflux condenser, a stirrer, a thermometer, and a nitrogen inlet; the above reaction system was heated to 70°C, and then the catalyst solution prepared above was added dropwise to a reaction system while stirring (the use amount of catalyst is 0.0155% of IPDI mass); a reaction temperature was controlled between 70°C and 80°C for a polymerization reaction; and when the NCO mass content of the reaction system is 23%, p-toluenesulfonic acid with the same molar amount as the catalyst was added to terminate the reaction.

A thin film evaporator was used to evaporate and remove unreacted isophorone diisocyanate monomers from a polymerization reaction solution under conditions of a temperature of 200°C and an absolute pressure of 100 Pa, with a staying time of 10 minutes, so that a content was 0.3wt%; and an IPDI trimer (i.e. isocyanate having a structure B) with a mass content of 58% was dissolved in solvent oil S100 to obtain a solution product with a solid content of 70wt%, and a polyisocyanate composition solution 4 was obtained.

**Example 5**

Preparation of catalyst solution: benzyltrimethylammonium hydroxide was dissolved in isooctanol to prepare a solution with a concentration of 5wt%.

1000 g of IPDI (a mass content of methyl-substituted IPDI being 0.55%) was placed in a round-bottom flask equipped with a reflux condenser, a stirrer, a thermometer, and a nitrogen inlet; the above reaction system was heated to 90°C, and then the catalyst solution prepared above was added dropwise to a reaction system while stirring (the use amount of catalyst is 0.016% of IPDI mass); a reaction temperature was controlled between 100°C and 110°C for a polymerization reaction; and when the NCO mass content of the reaction system is 26%, the system was heated to 130°C (a heating rate of 1°C/min), and after heating, staying was performed for 30 minutes to obtain the polymerization reaction solution.

A thin film evaporator was used to evaporate and remove unreacted isophorone diisocyanate monomers from a polymerization reaction solution under conditions of a temperature of 190°C and an absolute pressure of 50 Pa, with a staying time of 10 minutes, so that a content was 0.3wt%; and an IPDI trimer (i.e. isocyanate having a structure B) with a mass content of 64% was dissolved in butyl acetate to obtain a solution product with a solid content of 70wt%, and a polyisocyanate composition solution 5 was obtained.

**Comparative Example 1**

Preparation of catalyst solution: the same as Example 3

1000 g of IPDI (a mass content of methyl-substituted IPDI being 0.002%) was placed in a round-bottom flask equipped with a reflux condenser, a stirrer, a thermometer, and a nitrogen inlet; the above reaction system was heated to 70°C, and then the catalyst solution prepared above was added dropwise to a reaction system while stirring (the use amount of catalyst is 0.015% of IPDI mass); a reaction temperature was controlled between 100°C and 110°C for a polymerization reaction; and when the NCO mass content of the reaction system is 26%, dibutyl phosphate with the same molar amount as the tetrabutylammonium difluoride was added to terminate the reaction, so as to obtain a polymerization reaction solution.

A thin film evaporator was used to evaporate and remove unreacted isophorone diisocyanate monomers from a polymerization reaction solution under conditions of a temperature of 200°C and an absolute pressure of 100 Pa, so that a content was 0.33wt%; and an IPDI trimer (i.e. isocyanate having a structure B) with a mass content of 64% was dissolved in butyl acetate to obtain a solution product with a solid content of 70%, and a polyisocyanate composition 3-1 was obtained.

**Comparative Example 2**

Preparation of catalyst solution: the same as Example 3

1000 g of IPDI (a mass content of methyl-substituted IPDI being 0.60%) was placed in a round-bottom flask equipped with a reflux condenser, a stirrer, a thermometer, and a nitrogen inlet; the above reaction system was heated to 70°C, and then the catalyst solution prepared above was added dropwise to a reaction system while stirring (the use amount of catalyst is 0.015% of IPDI mass); a reaction temperature was controlled between 70°C and 80°C for a polymerization reaction; and when the NCO mass content of the reaction system is 25%, dibutyl phosphate with the same molar amount as the tetrabutylammonium difluoride was added to terminate the reaction, so as to obtain a polymerization reaction solution.

A thin film evaporator was used to evaporate and remove unreacted isophorone diisocyanate monomers from a polymerization reaction solution under conditions of a temperature of 200°C and an absolute pressure of 100 Pa, so that a content was 0.33wt%; and an IPDI trimer (i.e. isocyanate having a structure B) with a mass content of 62% was dissolved in butyl acetate to obtain a solution product with a solid content of 70%, and a polyisocyanate composition 3-2 was obtained.

The components and properties of the various polyisocyanate compositions prepared in the above examples and comparative examples were tested, and the results are shown in Table 1.

Table 1 Component and Storage Stability Test Results of Polyisocyanate Compositions

| Polyisocyanate composition solution | Methyl-substituted IPDI content/% | Molar ratio of a structure A to a structure B | NCO content/% | Monomer content/% | S100 solvent oil dissolution turbidity/NTU | Dissolution viscosity of butyl acetate at 25°C, cP |
|---|---|---|---|---|---|---|
| Example 1 | 0.01 | 0.0005 | 12.4 | 0.33 | 0.8 | 600 |
| Example 2 | 0.08 | 0.005 | 12.1 | 0.31 | 0.6 | 650 |
| Example 3 | 0.18 | 0.01 | 12.0 | 0.32 | 0.5 | 700 |
| Example 4 | 0.35 | 0.02 | 11.8 | 0.32 | 0.3 | 800 |
| Example 5 | 0.55 | 0.03 | 12.0 | 0.32 | 0.2 | 1000 |
| Comparative Example 1 | 0.002 | 0.0001 | 12.2 | 0.32 | 1 | 550 |
| Comparative Example 2 | 0.60 | 0.033 | 11.9 | 0.32 | 0.1 | 1200 |

The above results indicate that the solubility of the non-polar solvent can be enhanced by controlling the molar ratio of isocyanurate having the structure A to isocyanurate having the structure B, while avoiding viscosity increase in downstream construction.

Although the present application has been described in detail for illustrative purposes in the foregoing description, these are for illustrative purposes only and changes may be made therein by those skilled in the art without departing from the spirit and scope of the present application, the scope of which is defined by the claims.

## Claims

1. A polyisocyanate composition, comprising isocyanurate having a structure A and isocyanurate having a structure B,

wherein the structure A is:

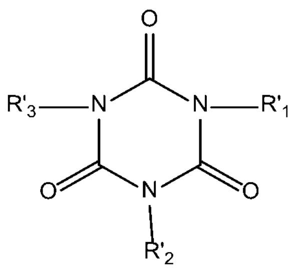

(A)

R'$_1$, R'$_2$ and R'$_3$ are respectively selected from the following four structures, wherein at least one of R'$_1$, R'$_2$ and R'$_3$ is selected from a structure (I) or a structure (II):

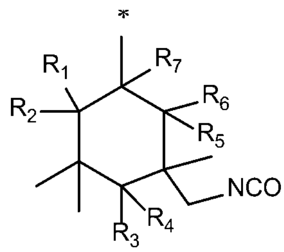

(I),

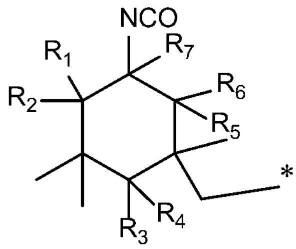

(II),

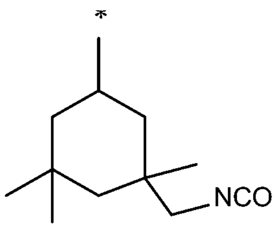

(III),

and

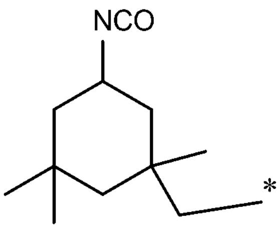

(IV);

one, two, or three group(s) among $R_1$, $R_2$, $R_3$, $R_4$, $R_5$, $R_6$ and $R_7$ in the above structural formulae is/are $CH_3$, and the rest are hydrogen;
the structure B is

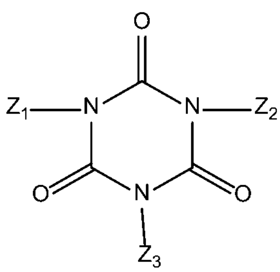

,

$Z_1$, $Z_2$ and $Z_3$ may be any one or both of the following two structures:

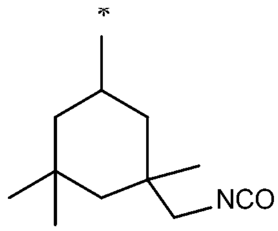

(III),

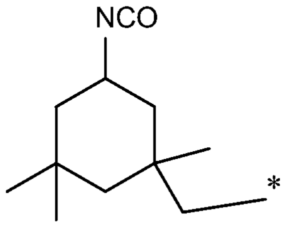

(IV);

and
a molar ratio of the isocyanurate having the structure A to the isocyanurate having the structure B is in a range from 0.0005 to 0.03.

2. The polyisocyanate composition according to claim 1, wherein the molar ratio of the isocyanurate having the structure A to the isocyanurate having the structure B is in a range from 0.005 to 0.02.

3. The polyisocyanate composition according to claim 1 or 2, wherein a mass content of the isocyanurate having the structure B is in a range from 50% to 75%, preferably in a range from 50% to 70%, more preferably in a range from 55% to 65%, and more preferably in a range from 59% to 63%.

4. The polyisocyanate composition according to any one of claims 1 to 3, wherein a mass content of isocyanate groups is in a range from 16% to 18%, preferably in a range from 16.5% to 17.5%.

5. The polyisocyanate composition according to any one of claims 1 to 4, wherein the polyisocyanate composition is dissolved in a solvent to obtain a polyisocyanate composition solution, and the solvent is selected from any one or a combination of at least two of butyl acetate, ethyl acetate, solvent oil, toluene, xylene, propylene glycol methyl ether acetate, and 2-heptanone.

6. The polyisocyanate composition according to claim 5, wherein a mass proportion of the solvent is in a range from 28% to 32%, based on 100% of total mass of the polyisocyanate composition solution.

7. The polyisocyanate composition according to claim 5 or 6, wherein the polyisocyanate composition solution corresponding to a solution state obtained by dissolving the polyisocyanate composition in the solvent has an NCO group mass content ranging from 11.2% to 12.8%, based on 100% of the total mass of the polyisocyanate composition solution.

8. The polyisocyanate composition according to any one of claims 1 to 7, wherein a content of an isophorone diisocyanate monomer is less than 0.5wt%, preferably less than 0.35wt%, and more preferably less than 0.1wt%.

**INTERNATIONAL SEARCH REPORT**

International application No.
**PCT/CN2023/118777**

**A. CLASSIFICATION OF SUBJECT MATTER**

C08G18/79(2006.01)i

According to International Patent Classification (IPC) or to both national classification and IPC

**B. FIELDS SEARCHED**

Minimum documentation searched (classification system followed by classification symbols)

IPC:C08G18

Documentation searched other than minimum documentation to the extent that such documents are included in the fields searched

Electronic data base consulted during the international search (name of data base and, where practicable, search terms used)

CNTXT, ENTXTC, ENTXT, STN, CNKI, DWPI, ISI, bing, 百度学术, BAIDU SCHOLAR: 万华化学, 刘伟, 任一臻, 林成栋, 石滨, 尚永华, 杨智民, 张现锋, 谭军, 乔小飞, 黎源, 异佛尔酮, 异弗尔酮, 二异氰酸酯, 甲基, 甲基化, 甲基接枝, 甲基取代, 三聚体, 四甲基, 五甲基, 六甲基, 环己, 异氰酸, IPDI, 溶解性, 油溶性, 脂溶性, 粘度, 黏度, 氰化氢, 光气, +isophorone+, +diisocyanate+, methyl+, methylat+, trimer+, +isocyan+, +cyclohex+, +tetramethyl+, +pentamethyl+, +hexamethyl+, solubility, viscosity

**C. DOCUMENTS CONSIDERED TO BE RELEVANT**

| Category* | Citation of document, with indication, where appropriate, of the relevant passages | Relevant to claim No. |
|---|---|---|
| A | CN 1699352 A (SOUTHEAST UNIVERSITY) 23 November 2005 (2005-11-23) description, page 1, paragraph 2 to page 3, paragraph 2 | 1-8 |
| A | CN 115894857 A (WANHUA CHEMICAL GROUP CO., LTD.) 04 April 2023 (2023-04-04) description, paragraphs [0005]-[0016] | 1-8 |
| A | MARIA WEJCHAN-JUDEK et al. "Trimerization of 3-isocyanatomethyl-3,5,5-trimethylcyclohexyl isocyanate (IPDI)" *Polimery*, Vol. 46, No. 2, 31 December 2001 (2001-12-31), pages 131-132 | 1-8 |
| A | US 2018327538 A1 (EVONIK DEGUSSA GMBH) 15 November 2018 (2018-11-15) embodiments | 1-8 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 March 2024** | **19 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/118777**

| Patent document cited in search report | Publication date (day/month/year) | Patent family member(s) | Publication date (day/month/year) |
|---|---|---|---|
| CN 1699352 A | 23 November 2005 | CN 1283631 C | 08 November 2006 |
| CN 115894857 A | 04 April 2023 | None | |
| US 2018327538 A1 | 15 November 2018 | EP 3401344 A1 | 14 November 2018 |
| | | EP 3401344 B1 | 08 April 2020 |
| | | JP 2018188439 A | 29 November 2018 |
| | | JP 7287758 B2 | 06 June 2023 |
| | | US 10793664 B2 | 06 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004176562 A1
- US 6093817 A
- CN 107827832
- CN 115894857 A